# EUROPEAN PATENT SPECIFICATION

(11) **EP 1 465 565 B1**
(45) Date of publication and mention of the grant of the patent: **24.10.2012**
(21) Application number: 02793623.6
(22) Date of filing: 11.12.2002
(51) Int. Cl.: A61F 13/15, A61F 13/514, A61F 13/535, A61F 5/44, A41B 13/04

(54) **ABSORBENT ARTICLE**
ABSORBIERENDER ARTIKEL
ARTICLE ABSORBANT

(30) Priority: 28.12.2001 SE 0104438
(43) Date of publication of application: 13.10.2004
(73) Proprietor: SCA Hygiene Products AB, 405 03 Göteborg (SE)
(72) Inventor: HERMANSSON, Kent, S-426 54 Västra Frölunda (SE); PORSÖ, Berith, S-433 30 Partille (SE)
(74) Representative: Olsson, Stefan
(86) International application number: PCT/SE2002/002287
(87) International publication number: WO 2003/059223

(56) References cited:
- EP-A1- 0 560 630
- WO-A1-01/13851
- WO-A1-01/15898
- SE-A- 9 904 202
- US-A- 2001 000 529

## Description

### TECHNICAL FIELD

The present invention relates to an absorbent article in form of non-refastenable absorbent pants, such as incontinence pants, nappy pants or sanitary towel pants, said absorbent article has a longitudinal direction and a transverse direction and comprising an elastic waist portion, an absorbent element, which has a length and a width and is intended to at least cover the genitals of the wearer during use of the article, and a liquid-impermeable outer layer which is intended to enclose the absorbent element on at least that side thereof which faces away from the wearer during use of the article. The invention also relates to a method of manufacturing such absorbent articles of pants-like shape.

### BACKGROUND ART

Absorbent disposable products for taking up urine, faeces or menstrual blood have developed greatly since they came into more general use during the 1960s and 1970s. As they are disposable products, it is necessary that they can be manufactured and sold at a very low price. At the same time, it is important that the products function well and reliably. Good fit and comfort are also important characteristics. The first disposable nappies were made of two-part products, consisting of an outer pant made of plastic, intended to be re-used, and a rectangular absorbent insert for disposable use. The absorbent material in these inserts initially consisted of cellulose tissue. Later, better absorption materials made of what is known as fluff pulp made of cellulose were developed. The fit and comfort of these early nappies were poor. The products were unwieldy and uncomfortable for the wearer. Towards the end of the 1970s, the first complete disposable nappies arrived, that is to say nappies in which the absorption bodies were integrated with a liquid-impermeable outer layer. The absorption materials have developed and improved, which has resulted in the possibility of the absorption bodies being adapted better to the anatomy of the wearer. Hourglass-shaped absorption bodies with a narrower crotch portion between the two end portions are now predominant. The trend has also been towards increasingly thin products, which has been made possible by the inclusion of what are known as superabsorbent materials in the absorption body. There are many reasons why thinner and even smaller absorption bodies are desirable. A thinner, smaller absorption body is more comfortable and more discreet, which is especially important for adult incontinent wearers. A reduction in volume is also very important financially because the product then requires less storage space and is easier to transport and takes up less shelf space in shops. This is important for the financial management of the shops, and if a manufacturer can produce products requiring less space in the shops than the products of the competitors, this affords a not inconsiderable competitive advantage. Moreover, there is increased pressure from authorities, in particular as far as disposable articles are concerned, to use as little material as possible for the purpose of reducing the burden on the environment.

The smaller the absorption bodies become, the more important it becomes that the absorption bodies come to lie in the correct place directly in front of the genitals of the wearer and remain in place during use even when the wearer is very active and moves a great deal. The demands of consumers for discretion, comfort and reliable functioning are also increasingly exacting. Requirements for the absorption body to come to lie correctly when put on and then be retained in the correct place have therefore increased the need for good fixing of the article on the body and the need for very good adaptability to the body when the wearer moves, at the same time as requirements have increased for the article always to come to lie in the correct place when the article is put on the wearer. This has led to the development of what are known as nappy pants, which have elastic portions for improved fit and comfort and increased flexibility during movements of the wearer compared with conventional absorbent articles.

An early patent publication relating to nappy pants of the disposable type is GB 2 112 267-A. However, this publication from 1983 discloses primitive nappy pants which did not become a commercial product. Not until the 1990s did absorbent articles of pants-like shape and construction become a major commercial product. Pants-like articles now exist in the form of nappies for infants and nappies for adults and to some extent sanitary towel pants for absorption of menstrual fluid. Previously commercially available nappy pants have been designed in principle like conventional nappies with a front portion and a rear portion and also an intermediate crotch portion, the front and rear portions being interconnected by a side seam between each leg opening and the waist opening of the pants. The nappy pants have been produced by plane nappy-like pieces being produced in a continuous web, the individual nappy pants being formed by nappy-like blanks being folded double and provided with said side seams to form nappy pants. These side seams project laterally from the finished product and are undesirable because they project and interfere with the fit of garments worn over the top. On account of their shape, they are visible through garments worn on top of the nappy pants and fitting closely around the body. They can also snag in clothing and even cause tears in nylon tights. Such projecting side seams can also chafe and give rise to pressure sores on wearers who spend a lot of time lying on their side.

WO 00/61049 has proposed improved nappy pants, in which the projecting sides seams have been eliminated. In this construction, the side seams have been eliminated by virtue of elastic side portions extending continuously in one piece from the front portion of the nappy pants to their rear portion on both sides of the nappy pants. However, the nappy pants according to WO 00/61049 have a number of disadvantages. The nappy pants according to said publication have what is referred to as a chassis, which extends over the entire nappy pants and forms the front portion, the crotch portion and the rear portion and is most reminiscent of a conventional nappy, and also said elastic side panels which each overlap the chassis at both the front and the rear on the nappy pants. These overlapping portions do not serve any actual purpose on the finished product and are in fact undesirable because a lot of material is wasted, that is to say they are used for no other purpose than joining together. The overlapping, joined-together portions are less of a nuisance and less uncomfortable for the wearer than the projecting side seams on previously known nappy pants. Owing to their unfavourable positioning, particularly at the rear, the overlapping portions can still give rise to a risk of chafing and back sores on wearers who spend a lot of time lying on their back. Another disadvantage of the nappy pants according to WO 00/61049 is that said chassis is relatively large and, as this portion is relatively rigid at least in comparison with the elastic side portions of the nappy pants, the nappy pants as a whole are not as adaptable to the body of the wearer but there is a risk that the nappy pants will be displaced from their optimum position in relation to the body when a wearer lies in bed and moves. In particular the rigid rear portion of the nappy pants can be displaced and pull both the crotch portion and the front portion from their optimum positions directly in front of the genitals of the wearer because the front and rear portion are essentially rigidly joined together in one piece with the crotch portion.

A problem with absorbent articles of pants-like shape is that conventional articles of this kind tightly enclose the area around the genitals of the wearer, Whereby this area, when body fluids are excreted, becomes locally saturated with moisture and vapour, which has an adverse effect on the skin of the wearer. In some cases disturbing skin irritation can occur. Furthermore, also minor chafing of the article against the skin of the wearer, in combination with a moist environment and during lengthy usage of the article, can give rise to sores on older people with flesh that heals poorly.

Breathable and vapour-permeable but liquid-impermeable materials for use in absorbent articles, such as nappies, incontinence products or sanitary towels, are previously known. Breathable materials can consist of perforated plastic films, as described in US 5,628,737, or of micro-porous plastic films, as described in, for example, EP-A-0238200. Another example of breathable materials are so-called non-woven materials which are air-permeable and vapour-permeable and which, if required, have been treated with means to improve the liquid-impermeability, as is described in EP-A-0196654. In absorbent articles of pants-like shape, with a fit which tightly encloses around the wearer, the need for air-permeability and vapour-permeability is important. A further problem is that urine and other body fluids give rise to evil-smelling substances, such as ammonia and amines, which also cause a rise of pH. At high pH, the equilibrium is shifted for many smelly substances in such a manner that more volatile components are formed, and that, for this reason, they smell stronger than at a low pH.

An essential problem with absorbent articles of pants-like shape is that the demand for absorbent material is greatest where the space is the least, i.e. in the area directly in front of the genitals of the wearer. This in combination with all the above-mentioned problems associated with absorbent articles of pants-like shape has resulted in a great need to find a pants-like article which functions well in all respects and which, in addition, it is possible to manufacture rationally.

### DISCLOSURE OF INVENTION

By means of the present invention, an improved absorbent article of pants-like shape has been produced.

An article of the type referred to in the introduction is to this end characterized in that said elastic waist portion is made from an elastic first piece which has a length and a width and which is elongate in a length direction parallel to said transverse direction and is intended in use to partly surround the trunk of the wearer and to form a rear portion and side portions of the pants-like article, in that a second piece forming part of the article is designed to form a front portion and a crotch portion of the pants-like article, in that said second piece has a length and a width and is elongate along the longitudinal direction with two opposite end edges and two opposite longitudinal side edges, in that the width of the second piece is, at least in the crotch portion, smaller than the length of the first piece, in that the second piece is arranged with its length direction at right angles in relation to the length direction of the first piece and is connected by a first end portion to one edge portion along the length direction of the first piece, centrally thereon, in that one end portion of the first piece is connected to a first side edge portion of one of said side edges of the second piece, and in that the other end portion of the first piece is connected in a corresponding manner to a second side edge portion of the other of said side edges of the second piece, in addition to which the absorbent element is in its entirety arranged on the second piece, in that the width of the absorbent element is narrowest in an area in the crotch portion, which area during use of the article is intended to be situated substantially in front of the genitals of the wearer, in that the absorbent element is narrowing in a direction towards said area, and in that the absorption capacity of the absorbent element per unit area is greatest in the narrowest area.

By virtue of the fact that the entire rear portion as well is elastic and, together with the elastic side portions, forms a single continuous elastic first piece, the pants as a whole are more adaptable to body movements. Local irregularities are taken up and smoothed out by the continuous elastic piece and are not transferred to the more rigid parts of the front portion and crotch portion located directly in front of the genitals of the wearer. Compared with conventional nappies and previously known absorbent articles of pants-like shape, the article according to the present invention affords superior fit and comfort. Most of the pants-like garment is completely smooth. The seams required are arranged at the transition between the front portion and the first piece and at the transition between the first and the second piece in the crotch portion. These seams come to lie in places which are not subjected to any significant pressure during use of the article, and there is less risk of chafing and pressure sores caused by seams on the pants-like article. The risk of leakage diminishes, at the same time as the capacity of the absorption material is exploited in an efficient manner, due to the fact that the article has been optimised in such a manner that the absorption capacity is greatest where the demand is greatest, i.e. in the area directly in front of the genitals of the wearer.

The design of the absorbent article according to the invention in only two part pieces, where the absorbent element is in its entirety located on the second part piece, affords greater freedom of choice with regard to manufacturing method compared with previously known articles of pants-like shape.

According to a suitable embodiment, the invention is characterized in that said absorption capacity per unit area for the absorbent element increases in a direction towards the narrowest area. According to another embodiment, the invention is furthermore characterized in that said absorption capacity per unit area for the absorbent element increases continuously in said direction.

According to another suitable embodiment, the invention is characterized in that the absorbent element comprises superabsorbent material, and in that the absorption capacity of the absorbent element is controlled by the content of the superabsorbent material.

The term absorption capacity per unit area here denotes the absorption capacity across the product over one unit area, i.e. the absorption capacity in different areas is controlled by the absorption capacity in g per unit volume in a product of uniform thickness. Alternatively, the absorption capacity in different areas may be varied by varying of the thickness. Furthermore, the absorption capacity in g per unit volume as well as the thickness may be varied in different areas. '

According to another embodiment, the invention is characterized in that the length of the second piece is greater than the width of the first piece, and in that the projecting portion of the second piece formed by the length difference is in its entirety located below that side edge of the first piece which is the lower one during use of the article, and there forms the crotch portion. According to a further embodiment, the invention is in this connection characterized in that said projecting portion has a smaller width than the remainder of the second piece.

According to another embodiment, the invention is characterized in that the absorbent element extends in its length direction over the entire crotch portion and a little way up over the front portion in the direction of that side edge of the second piece which is the upper one during use of the article. According to a further embodiment, the invention is in this connection characterized in that the absorbent element forms a concave shape,with its side edge portions in the crotch portion.

According to another embodiment, the invention is characterized in that the absorbent element is arranged so as to extend with its lower end portion only a little way over the crotch portion and over less than half the extent of said projecting portion.

According to one embodiment, the invention is characterized in that said connected side edge portions and end portions of said first and second pieces are arranged in an overlapping manner, with the inside of an overlapping portion being arranged against the outside of an overlapped portion.

According to another embodiment, the invention is characterized in that the second piece includes a liquid-permeable inner layer and said liquid-impermeable outer layer, and in that the absorbent element is arranged between said inner and outer layers, the inner and outer layers extending in the lateral direction and longitudinal direction outside the absorbent element and being interconnected there.

According to one embodiment, the invention is characterized in that the liquid-impermeable layer only covers the absorption body.

According to one embodiment, the rest of the second piece (2) is breathable and vapour-permeable.

According to a further embodiment, the first piece is rectangular, which simplifies the manufacturing.

According to one embodiment, the second piece has a concave portion between the first piece and up to said side edge portions of the second piece, wherein the concave portion forms cut-outs for the legs of the wearer. According to another embodiment, the second piece is, at least along a part of its length, narrowing towards the first piece, wherein the narrowing portion forms cut-outs for the legs of the wearer.

According to one embodiment, the invention is characterized in that the second piece is essentially inelastic. The term inelastic here denotes that the front section, under normally occurring stresses, does not stretch.

According to another embodiment, the invention is characterized in that the elastic members are arranged along the edges of the second piece, along at least the crotch portion for the forming of leg elastics, intended to lie in sealing contact with the legs of the wearer. According to one embodiment, the invention is further characterized in that the elastic members extend in and from an area in the rear portion where the first and the second piece overlap each other and further along the crotch portion along the edges of the second piece in a direction towards the front portion.

In such a design, the leg elastic is connected with the waist elastic in the first piece, which gives the pants-like article a good fit, and sealing contact around the absorbent element.

According to a suitable embodiment, the invention is characterized in that said superabsorbent material, entirely or partially, consists of a partially neutralised superabsorbent material for the regulation of pH in the environment around the skin of the wearer, wherein a pH regulating superabsorbent material, which after wetting accomplishes a pH in the absorbent article in the interval of 3.5-4.9, preferably 4.1-4.7, is chosen.

According to a further embodiment, the invention is characterized in that said superabsorbent material, entirely or partially, consists of a superabsorbent material which is not neutralised or is neutralised to a low degree, preferably with a neutralisation degree of between 0-19%. A superabsorbent material which is not neutralised or is neutralised to a low degree is stickier than other superabsorbent materials and therefore adheres better in a fibre matrix, which in turn means that a larger amount of superabsorbent material can be admixed in an absorbent structure, compared to superabsorbents which are less sticky.

A method of manufacturing an article of the type mentioned in the introduction is characterized according to the invention in that said elastic waist portion is made from an elastic first piece which is essentially rectangular with a length and a widthand is intended in use to partly surround the trunk of the wearer and to form a rear portion and side portions of the pants-like article, in that a second piece forming part of the article is designed to form a front portion and a crotch portion of the pants-like article, in that said second piece is made with a length and a width and is arranged with its length direction along the longitudinal direction with two opposite end edges and two opposite longitudinal side edges, in that the width of the second piece is designed, at least in the crotch portion, smaller than the length of the first piece, in that the second piece is arranged with its length direction at right angles in relation to the length direction of the first piece and is connected by a first end portion to one edge portion along the length of the first piece, centrally thereon, in that one end portion of the first piece is connected to a first side edge portion of one of said side edges of the second piece, and in that the other end portion of the first piece is connected in a corresponding manner to a second side edge portion of the other of said side edges of the second piece, and in addition to which the superabsorbent material is arranged in the absorbent element, in that the absorbent element is shaped with a width which is narrowest in an area in the crotch portion, which area during use of the article is intended to be situated substantially in front of the genitals of the wearer, in that the absorbent element is made with a continuously narrowing width in a direction towards said area, in that superabsorbent material is arranged in the absorbent element with a continuously increasing concentration in the same direction, and in that the absorbent element is in its entirety arranged on the second piece forming part of the article before said piece is connected to said first piece.

### DESCRIPTION OF DRAWINGS

The invention will be described in greater detail below with reference to illustrative embodiments shown in the accompanying drawings, in which
- Fig. 1: shows schematically a phase of the construction of an absorbent article according to a first embodiment.
- Fig. 2: shows schematically in plane form an assembled article according to the first embodiment.
- Fig. 3: shows the article according to Fig. 2 in perspective.
- Fig. 4: shows a section along the line IV-IV in Fig. 1.
- Fig. 5: shows schematically a phase of the construction of an absorbent article according to a second embodiment.
- Fig. 6: shows in plane form an assembled article according to the second embodiment.
- Fig. 7: shows in plane form an assembled article according to a third embodiment.
- Fig. 8: shows in plane view an assembled article according to a fourth embodiment of the invention.
- Fig. 9: shows schematically the distribution of superabsorbent material in one embodiment of an absorbent element belonging to the article.
- Fig. 10: shows a section along the line X-X in Fig. 8.

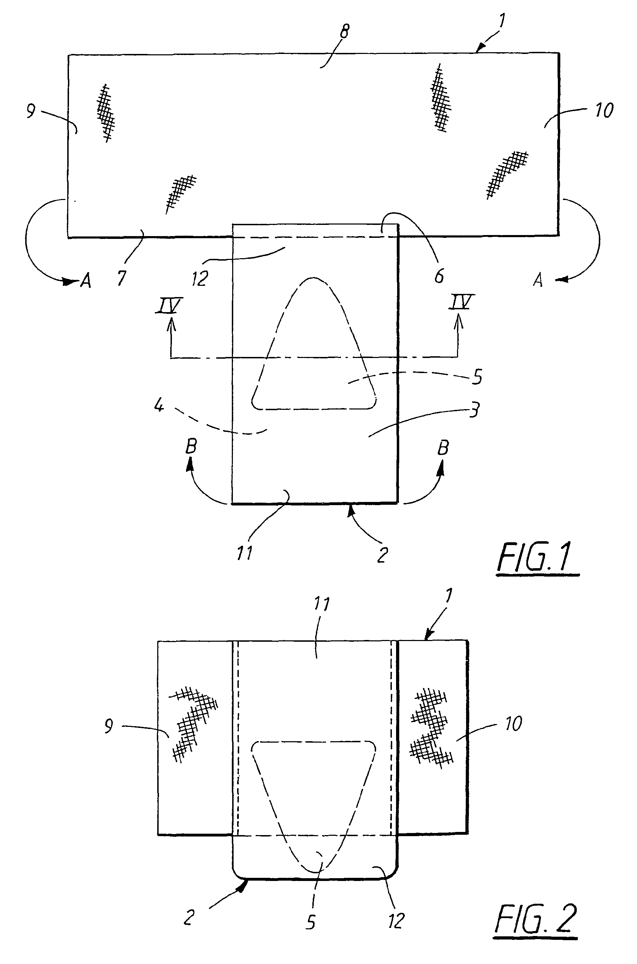

As can be seen from Fig. 1, the article in the embodiment according to Fig. 1 comprises a first piece 1. This piece is elastically stretchable and is shown in Fig. 1 in a plane and even extended state, in which the elastic piece 1 is essentially rectangular. The elastic first piece 1 can be made from conventional materials well-known to the person skilled in the art, such as woven elastic materials, elastic non-wovens, elastic laminates or elastic films. Another alternative is a material with prestressed elastic threads arranged on a supporting material, for example non-woven. The important factor is that the elastic piece is stretchable for a wearer when the article is put on, and that it fits closely with suitable tightness around the wearer during use of the article.

The tightness is of course regulated by means of size and elastic stretchability.

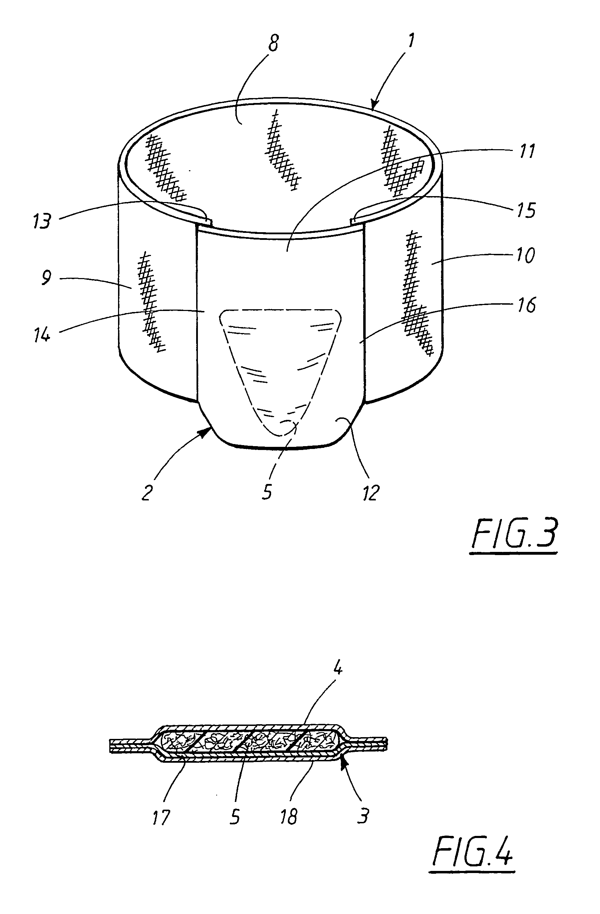

The article includes a second piece 2 which, as can be seen from Figs. 1 and 4, consists of an outer layer 3, an inner layer 4 and an absorbent element 5 arranged between these- In the embodiment in plane form shown in Fig. 1, this element is essentially triangular. The material selected for the absorption element is important. It is especially important that the absorbent element 5 is designed with high absorption capacity where the demand is greatest, namely in the area around the genitals of the wearer. This has been solved according to the invention, due to the fact that the absorbent element comprises superabsorbent material, and due to the fact that concentration of the superabsorbent material in the form of particles or fibres is greatest in the area which during use of the article is situated directly in front of the genitals of the wearer. In the shown illustrative embodiment the concentration of superabsorbent material increases continuously in a direction towards the narrow end of the absorbent element 2. The absorbent element, moreover, can be chosen from among materials or material combinations well-known to the person skilled in the art. For example, the absorbent element can consist of cellulose fluff pulp with superabsorbent materials in powder or fibre form added in. The absorbent element can also consist of a foam material with an admixture of superabsorbent material.

The presence of urine and other body fluids close to the skin of the wearer gives rise to growth of bacteria which constitutes a risk for infections and the emergence of odours. For this reason, it may be suitable to entirely or partially use superabsorbent material consisting of a partially neutralised superabsorbent material for the regulation of pH in the environment around the skin of the wearer. In this connection, a pH regulating superabsorbent material, which after wetting accomplishes a pH in the absorbent article in the interval of 3.5-4.9, preferably 4.1-4.7, is chosen. The regulation of pH is described in detail in the Swedish patent 511 838 C2, and in the Swedish patent 513 374 C2, which are include as reference.

Another suitable superabsorbent material may consist of a superabsorbent material which is not neutralised or is neutralised to a low degree, preferably with a neutralisation degree of between 0-19%. A superabsorbent material which is not neutralised or is neutralised to a low degree is stickier than other superabsorbent materials and therefore adheres better in a fibre matrix, which in turn means that a larger amount of superabsorbent material can be admixed in an absorbent structure, compared to superabsorbents which are less sticky.

It is not required that the superabsorbent material is pH regulating, but it is in the absorbent element that a pH interval of 3.5-4.9 is accomplished after wetting. This may be accomplished by means of SAP, not neutralised, or neutralised SAP. It is also possible to accomplish a pH in said interval in the absorbent element after wetting by means of acidified pulp, or through addition of acidifying chemicals in the absorbent element.

The outer layer 3 is suitably liquid-impermeable and breathable. The breathable and liquid-impermeable layer 3 should have a so-called MTVR-value in the order of magnitude of 200-10000 g/m2/24h. The term MTVR stands for "Moisture Vapour Transmission Rate", i.e. the amount of vapour which can pass per unit area during a certain period of time.

The liquid-impermeable and breathable layer 3 can, for example, consist of a perforated polyethylene film, where the size of the holes has been chosen so that air and vapour may pass, but not liquid drops. If an absorbent article with a more textile-like appearance is desired, a liquid-impermeable film in combination with an outer fibre layer is suitable.

Breathable and vapour-permeable but liquid-impermeable materials for use in absorbent articles, such as napples, incontinence products and sanitary towels, are previously known in different embodiments. Breathable materials can, as mentioned above, consist of perforated plastic films. One example of such films is described in US 5,628,737. The liquid-impermeable and breathable outer layer 3 can also consist of micro-porous plastic films, as is described in, for example, EP-A-0238200. Another example of breathable materials are so-called non-woven which are air-permeable and vapour-permeable and which, if required, have been treated with means to improve the liquid-impermeability, as is described in EP-A-0196654. Breathability can be measured according to ASTM E96, where the equipment Lyssy has been used.

The inner layer 4 can be made from a liquid-permeable non-woven.

The second piece 2 is elongate and is arranged with its longitudinal direction at right angles in relation to the longitudinal direction of the first piece 1. The second piece 2 is connected by one end portion 6 to one longitudinal edge portion 7 of the first piece, centrally thereon. The connection can be made by means of, for example, adhesive, thermal bonding or ultrasonic bonding.

In Fig. 1, the arrows A and B illustrate how the first and second pieces are folded to form the absorbent article of pants-like shape shown in Figs. 2 and 3. The elastic piece 1 is folded in according to the arrows A shown to form the rear portion 8 and side portions 9, 10 of the article, while the second piece is folded upwards according to the arrows B to form the front portion 11 and crotch portion 12 of the article.

As can be seen most clearly from Fig. 3, one end portion 13 of the first piece is arranged so as to be overlapped a little by a first side edge portion 14 of the second piece, and the other end portion 15 of the first piece is in a corresponding manner arranged so as to be overlapped a little by a second side edge portion 16 of the second piece. These overlapping portions are interconnected by means of, for example, adhesive, thermal bonding or ultrasonic bonding.

In Fig. 4, the thickness of the second piece has been exaggerated for the sake of clarity. The figure shows an outer layer 3 consisting of a laminate of a plastic film 17 and a non-woven layer 18 arranged outside. The inner layer 4 can consist of a liquid-permeable non-woven of a type well-known to the person skilled in the art.

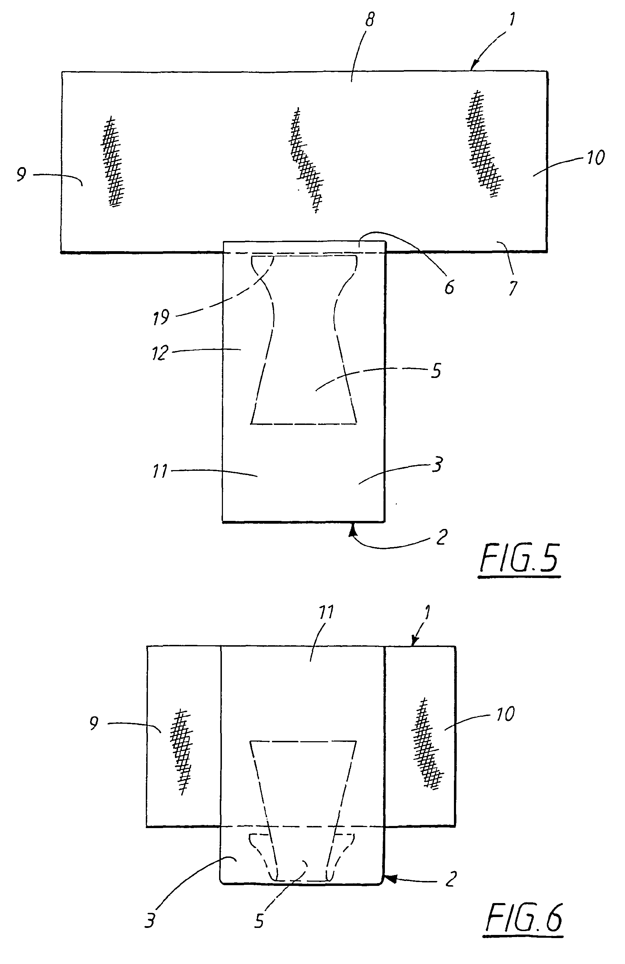

In the embodiment shown in Figs. 5 and 6, those parts corresponding to the same parts in the embodiment according to Figs. 1-4 have been provided with the same reference numbers. The only difference between the embodiment according to Figs. 5 and 6 and that described above is the shape and extent of the absorbent element 5. In this second embodiment, the absorbent element 5 is hourglass-shaped in plane form, as can be seen most clearly from Fig. 5, the narrower portion being intended to be arranged in the crotch of the wearer during use of the article. The upper end edge 19 of the absorbent element 5 is located directly adjacent to the lower edge portion 7 of the first elastic piece, which means that the absorbent element according to this second embodiment extends over the entire crotch area and a little way up over the front portion 11, as can be seen from Fig. 6. in this embodiment the concentration of superabsorbent material increases continuously from each direction in a direction towards the narrowest area of the absorbent element.

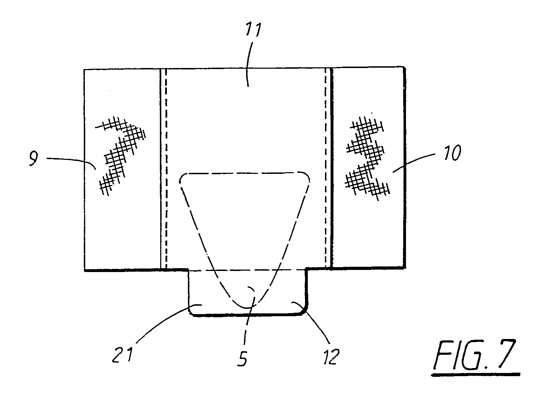

Fig. 7 shows a third embodiment which is slightly modified in relation to the first embodiment. The only difference is that a part piece 21 of the second piece is made narrower than the remainder of the second piece, in which way a pants-like article with a narrower crotch portion is obtained, as can be seen from Fig. 7.

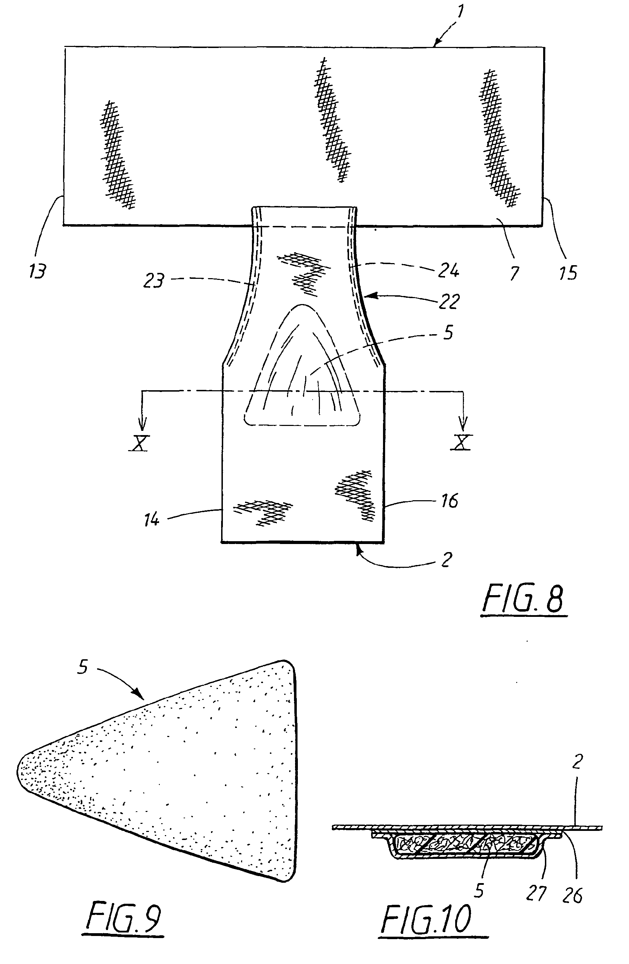

In the embodiment shown in Fig. 8, the parts corresponding to similar details in the other embodiments have been provided with the same reference numbers. The second piece 2 has a concave portion 22 which extends from the first piece 1 towards the side edge portions 14,16. These are, as in the above described embodiments, intended to be connected with the end portions 13 and 15 on the first piece 1.

The first piece 1 is formed of an elastic material and is intended during use of the article to achieve an elastic tightening around the waist of the wearer, which tightening keeps the article in place and gives it a good fit. In the embodiment shown in Fig. 8, the elastic members 23,24 are arranged along both sides of the concave portion 22 for the forming of leg elastic, which during use of the article is intended to be in sealing contact with the legs of the wearer. In the shown embodiment, the elastic members 23, 24 extend from the elastic first piece 1 to the side edge portions 14,16. The completed, assembled, pants-like article will thereby be elastically tight-fitting around both of the legs of the wearer. In this manner a good fit is obtained. If the elastic piece 1 and the elastic members 23,24 are formed so that they are elastically stretchable a longer distance, without the tension in the elastic piece 1 and in the elastic members 23,24 being increased to any considerable extent, the article can, without any annoying chafing, be used by wearers of different sizes. In such a design, the number of requisite sizes can be kept down, which leads to savings and rational manufacturing compared to if there is a need for a larger assortment of sizes.

Fig. 9 schematically shows the absorbent element 5 according to a suitable embodiment. In order to avoid giving rise to annoying chafing in the crotch of the wearer, the absorbent element 5 has been designed as narrowing in that direction which during use of the article is directed rearwards. A complication with this narrowing design is, as is mentioned above, that the absorbent element is narrowest in that portion which during use of the article is situated substantially in front of the genitals of the wearer, in which portion the demand for absorption capacity is greatest. To avoid the risk of leakage in the narrower portion of the absorbent element to the most practicable degree, the superabsorbent material in the form of particles or fibres; according to an embodiment of the invention, has been arranged in the absorbent material with an increasing concentration in said narrowing direction. This has been illustrated in Fig. 9 with the number of shown dots in correspondence with a gradient of the number of superabsorbent particles per unit volume of the absorbent element 5 in said direction.

In Fig. 4 an embodiment is shown where the liquid-impermeable but breathable outer layer 3 consists of a layer of the, second piece. According to one embodiment, the liquid-impermeable outer layer is arranged only over the absorbent element, and forms an outer layer of said element. In Fig. 10 a cross section along the line X-X in Fig. 8 is shown, in order to illustrate this. The absorbent element is here enclosed in a liquid-permeable layer 26, for example of non-woven, and of a liquid-impermeable and at the same time breathable layer 27, for example of a perforated plastic film. The two layers 26,27 extend beyond the absorbent element and a distance around it and are there interconnected. The unit formed by the absorbent element 5 and the two layers 26 and 27 is attached to the second piece 2 of the absorbent article, wherein the liquid-permeable layer 26 has been connected to the second piece 2, as is shown in Fig.10.

The invention is not limited to the illustrative embodiments described above, but a number of modifications are possible within the scope of the patent claims below.

The absorption unit shown in Fig. 10, consisting of the absorbent element 5 and the layers 26 and 27, may be replaced with an absorbent foam material in one piece, which foam material is provided with an integral liquid-impermeable and at the same time air-permeable layer.

When the expression liquid-impermeable and breathable has been used in the above text, it also implies that the material in question also is vapour-permeable, but that it does not allow passage of liquid drops.

In the above illustrative embodiments, as is evident from Fig. 3, the side edge portions 14,16 of the second piece have been arranged on the outside of the end edge portions 13,15 of the first piece. It is, of course, possible to do the reverse, i.e. with the end edge portions 13,15 of the first piece farthest out. These two alternatives are to be preferred, even though it is within the scope of the invention to obtain said connection by means of attaching the inside of each of the end edge portions of the first piece to the inside of each side edge portion of the second piece, or by means of connecting corresponding edge portions outside to outside.

The article according to the invention has turned out to be especially suitable as incontinence shields for men. Other applications, such as menstrual shields or incontinence shields for women, are also conceivable.

In the above described illustrative embodiments, the highest absorption capacity for the narrowest area, as measured across the absorbent element per unit area, has been achieved by means of varying the content of superabsorbent material. Another method of achieving higher absorption capacity in the narrowest area is to form the absorbent element with a higher basis weight in this area. This can, for example, be carried out on an absorbent element formed from a fluff pulp of cellulose. The absorption capacity for an absorbent element of a fluff pulp may also be controlled by varying the density in different portions.

The absorbent element can, of course within the scope of the invention, be formed from other materials, for example absorbent foam materials.

## Claims

1. Absorbent article in form of non-refastenable absorbent pants, such as incontinence pants, nappy pants or sanitary towel pants, said absorbent article has a longitudinal direction and a transverse direction and comprising an elastic waist portion, an absorbent element (5), which has a length and a width and is intended to at least cover the genitals of the wearer during use of the article, and a liquid-impermeable outer layer (3) which is intended to enclose the absorbent element on at least that side thereof which faces away from the wearer during use of the article,
**characterized in,**
**that** said elastic waist portion is made from an elastic first piece (1) which has a length and a width and which is elongate in a length direction parallel to said transverse direction and is intended in use to partly surround the trunk of the wearer and to form a rear portion (8) and side portions (9, 10) of the pants-like article,
in that a second piece (2) forming part of the article is designed to form a front portion (11) and a crotch portion (12) of the pants-like article, in that said second piece (2) has a length and a width and is elongate along the longitudinal direction with two opposite end edges and two opposite longitudinal side edges, in that the width of the second piece is, at least in the crotch portion (12), smaller than the length of the first piece (1), in that the second piece (2) is arranged with its length direction at right angles in relation to the length direction of the first piece and is connected by a first end portion (6) to one edge portion (7) along the length direction of the first piece, centrally thereon,
in that one end portion (13) of the first piece is connected to a first side edge portion (14) of one of said side edges of the second piece, and in that the other end portion (15) of the first piece is connected in a corresponding manner to a second side edge portion (16) of the other of said side edges of the second piece,
in addition to which the absorbent element (5) is in its entirety arranged on the second piece (2),
in that the width of the absorbent element (5) is narrowest in an area in the crotch portion (12), which area during use of the article is intended to be situated substantially in front of the genitals of the wearer, in that the absorbent element is narrowing in a direction towards said area, and in that the absorption capacity of the absorbent element per unit area is greatest in the narrowest area.

2. Absorbent article according to claim 1, **characterized in, that** said absorption capacity per unit area for the absorbent element (5) increases in a direction towards the narrowest area.

3. Absorbent article according to claim 1 or 2,
**characterized in,**
**that** said absorption capacity per unit area for the absorbent element (5) increases continuously in said direction.

4. Absorbent article according to any one of the preceding claims,
**characterized in, that** the absorbent element comprises superabsorbent material, and **in that** the absorption capacity of the absorbent element (5) is controlled by the content of the superabsorbent material.

5. Absorbent article according to any one of the preceding claims,
**characterized in,**
**that** the length of the second piece (2) is greater than the width of the first piece (1), and in that the projecting portion of the second piece formed by the length difference is in its entirety located below that side edge of the first piece which is the lower one during use of the article, and there forms the crotch portion (12).

6. Absorbent article according to claim 5, **characterized in, that** said projecting portion has a smaller width than the remainder of the second piece (2) (Fig. 7).

7. Absorbent element according to any one of the preceding claims,
**characterized in, that** the absorbent element (5) extends in its length direction over the entire crotch portion (12) and a little way up over the front portion (11) in the direction of that side edge of the second piece (2) which is the upper one during use of the article.

8. Absorbent article according to any one of claims 1-6,
**characterized in, that** the absorbent element (5) is arranged so as to extend with its lower end portion only a little way over the crotch portion (12) and over less than half the extent of said projecting portion (Fig. 2).

9. Absorbent article according to claim 7, **characterized in, that** the absorbent element (5) forms a concave shape with its side edge portions in the crotch area (Fig. 5).

10. Absorbent article according to any one of the preceding claims,
**characterized in,**
**that** said connected side edge portions (13, 15) and end portions (14, 16) of said first and second pieces (1 and, respectively, 2) are arranged in an overlapping manner, with the inside of an overlapping portion being arranged against the outside of an overlapped portion.

11. Absorbent article according to any one of the preceding claims,
**characterized in,**
**that** the second piece (2) includes a liquid-permeable inner layer (4) and said liquid-impermeable outer layer (3), and in that the absorbent element (5) is arranged between said inner and outer layers, the inner and outer layers extending in the lateral direction and longitudinal direction outside the absorbent element (5) and being interconnected there.

12. Absorbent article according to claim 11,
**characterized in, that** the liquid-impermeable outer layer (3) only covers the absorbent element.

13. Absorbent article according to claim 12,
**characterized in, that** the rest of the second piece (2) is breathable and vapour-permeable.

14. Absorbent article according to claim 11 or 12,
**characterized in, that** the liquid-impermeable outer layer (3) is breathable and vapour-permeable and that the rest of the article, over its entire area, is breathable and vapour-permeable.

15. Absorbent article according to any one of the preceding claims,
**characterized in,**
**that** the first piece (1) is rectangular.

16. Absorbent article according to any one of the preceding claims,
**characterized in,**
**that** the second piece (2) has a concave portion (22) between the first piece (1) and up to said side edge portions (14,16) of the second piece (2), wherein the concave portion forms cut-outs for the legs of the wearer.

17. Absorbent article according to any one of the preceding claims,
**characterized in,**
**that** the second piece (2) is, at least along a part of its length, narrowing towards the first piece (1), wherein the narrowing portion forms cut-outs for the legs of the wearer.

18. Absorbent article according to any one of the preceding claims,
**characterized in,**
**that** the second piece (2) is essentially inelastic.

19. Absorbent article according to any one of the preceding claims,
**characterized in,**
**that** elastic members (23,24) are arranged along the edges of the second piece (2), along at least the crotch portion for the forming of leg elastics, intended to lie in sealing contact with the legs of the wearer.

20. Absorbent article according to claim 19,
**characterized in, that** the elastic members (23,24) extend in and from an area in the rear portion where the first (1) and the second piece (2) overlap each other and further along the crotch portion along the edges of the second piece in a direction towards the front portion.

21. Absorbent article according to any one of claims 4-20,
**characterized in,**
**that** said absorbent element comprises a pH regulating material for regulation of pH in the environment around the skin of the wearer, wherein the pH regulating material is arranged to accomplish a pH in the interval of 3.5-4.9.

22. Method of manufacturing an absorbent article in form of non-refastenable absorbent pants, such as incontinence pants, nappy pants or sanitary towel pants, said absorbent article has a longitudinal direction and a transverse direction and comprising an elastic waist portion, an absorbent element (5), which has a length and a width and is intended to at least cover the genitals of the wearer during use of the article, and a liquid-impermeable outer layer (3) which is intended to enclose the absorbent element on at least that side thereof which faces away from the wearer during use of the article,
**characterized in,**
**that** said elastic waist portion is made from an elastic first piece (1) which is essentially rectangular with a length and a width and is intended in use to partly surround the trunk of the wearer and to form a rear portion (8) and side portions (9, 10) of the pants-like article,
in that a second piece (2) forming part of the article is designed to form a front portion (11) and a crotch portion (12) of the pants-like article, in that said second piece (2) is made with a length and a width and is arranged with its length direction along the longitudinal direction with two opposite end edges and two opposite longitudinal side edges, in that the width of the second piece (2) is designed, at least in the crotch portion (12), smaller than the length of the first piece (1),
in that the second piece is arranged with its length direction at right angles in relation to the length direction of the first piece and is connected by a first end portion (6) to one edge portion (7) along the length of the first piece, centrally thereon,
in that one end portion (13) of the first piece (1) is connected to a first side edge portion (14) of one of said side edges of the second piece (2), and in that the other end portion (15) of the first piece (1) is connected in a corresponding manner to a second side edge portion (16) of the other of said side edges of the second piece, and in addition to which superabsorbent material is arranged in the absorbent element (5),
in that the absorbent element (5) is shaped with a width which is narrowest in an area in the crotch portion (12), which area during use of the article is intended to be situated substantially in front of the genitals of the wearer,
in that the absorbent element is made with continuously narrowing width in a direction towards said area, in that superabsorbent material is arranged in the absorbent element with a continuously increasing concentration in the same direction, and in that the absorbent element (5) in its entirety is arranged on the second piece (2) forming part of the article before said piece is connected to said first piece (1).

## Patentansprüche

1. Absorbierender Artikel in Form einer nicht-wiederbefestigbaren absorbierenden Unterhose, wie z.B. einer Inkontinenzunterhose, einer Windelhose oder einer Bindeunterhose, wobei der absorbierende Artikel eine Längsrichtung und eine Querrichtung aufweist und einen elastischen Bauchteil, ein absorbierendes Element (5), das eine Länge und eine Breite aufweist und vorgesehen ist, um mindestens die Genitalien des Benutzers während der Anwendung des Artikels zu decken, und eine flüssigkeitsundurchlässige Außenschicht (3), der vorgesehen ist, um das absorbierende Element auf mindestens der Seite einzuschließen, die während der Anwendung des Artikels vom Benutzer abgewandt ist, umfasst
**dadurch gekennzeichnet,**
**dass** der elastische Bauchteil aus einem elastischen ersten Stück (1) hergestellt ist, das eine Länge und eine Breite aufweist und in einer Längsrichtung parallel zur Querrichtung gestreckt ist und vorgesehen ist, um während der Anwendung teilweise den Torso des Benutzers zu umgeben und einen Hinterteil (8) und Seitenteile (9, 10) des unterhosenähnlichen Artikels zu bilden,
**dass** ein zweites Stück (2), das einen Teil des Artikels bildet, ausgeformt ist, um einen Vorderteil (11) und einen Schrittteil (12) des unterhosenähnlichen Artikels zu bilden, dass das zweite Stück (2) eine Länge und eine Breite aufweist und entlang der Längsrichtung mit zwei gegenüberliegenden Endkanten und zwei gegenüberliegenden Längsseitenkanten gestreckt ist, dass die Breite des zweiten Stücks zumindest im Schrittteil (12) kleiner als die Länge des ersten Stücks (1) ist, dass das zweite Stück (2) mit seiner Längsrichtung in rechten Winkeln zur Längsrichtung des ersten Stücks angeordnet ist und an einem ersten Endteil (6) mit einem Kantteil (7) entlang der Längsrichtung des ersten Stücks, mittig darauf, verbunden ist,
**dass** ein Endteil (13) des ersten Stücks mit einem ersten Seitenkantteil (14) von einer der Seitenkanten des zweiten Stücks verbunden ist, und dass der andere Endteil (15) des ersten Stücks auf entsprechender Art und Weise mit einem zweiten Seitenkantteil (16) von der anderen der Seitenkanten des zweiten Stücks verbunden ist, wobei das absorbierende Element (5) darüber hinaus in seiner Gesamtheit auf dem zweiten Stück (2) angeordnet ist,
**dass** die Breite des absorbierenden Elements (5) in einem Bereich im Schrittteil (12) am schmalsten ist, welcher Bereich während der Anwendung des Artikels eingerichtet ist, um im Wesentlichen vor den Genitalien des Benutzers angeordnet zu sein, dass das absorbierende Element in einer Richtung gegen diesen Bereich schmaler wird, und dass die Absorptionskapazität des absorbierenden Elements pro Flächeneinheit im schmalsten Bereich am größten ist.

2. Absorbierender Artikel nach Anspruch 1, **dadurch gekennzeichnet, dass** die Absorptionskapazität des absorbierenden Elements (5) pro Flächeneinheit in Richtung gegen den schmalsten Bereich ansteigt.

3. Absorbierender Artikel nach Anspruch 1 oder 2, **dadurch gekennzeichnet, dass** die Absorptionskapazität des absorbierenden Elements (5) pro Flächeneinheit in dieser Richtung kontinuierlich zunimmt.

4. Absorbierender Artikel nach irgendeinem der vorhergehenden Ansprüche, **dadurch gekennzeichnet, dass** das absorbierende Element superabsorbierendes Material umfasst, und dass die Absorptionskapazität des absorbierenden Elements (5) durch den Inhalt vom superabsorbierenden Material reguliert wird.

5. Absorbierender Artikel nach irgendeinem der vorhergehenden Ansprüche, **dadurch gekennzeichnet, dass** die Länge des zweiten Stücks (2) größer als die Breite des ersten Stücks (1) ist, und dass der vorstehende Teil des zweiten Stücks, der durch den Längenunterschied gebildet ist, in seiner Gesamtheit unter der Seitenkante des ersten Stücks angeordnet ist, die die untere während der Anwendung des Artikels ist, und dort den Schrittteil (12) bildet.

6. Absorbierender Artikel nach Anspruch 5, **dadurch gekennzeichnet, dass** der vorstehende Teil eine kleinere Breite als der Rest des zweiten Stücks (2) aufweist (Fig. 7).

7. Absorbierendes Element nach irgendeinem der vorhergehenden Ansprüche, **dadurch gekennzeichnet, dass** sich das absorbierende Element (5) in seiner Längenrichtung über den ganzen Schrittteil (12) und ein Bisschen über den Vorderteil (11) hinauf in Richtung der Seitenkante des zweiten Stücks (2), die die obere während der Anwendung des Artikels ist, erstreckt.

8. Absorbierender Artikel nach irgendeinem der Ansprüche 1-6, **dadurch gekennzeichnet, dass** das absorbierende Element (5) angeordnet ist, um sich mit seinem unteren Endteil nur ein Bisschen über den Schrittteil (12) und über weniger als die Hälfte der Erstreckung des vorstehenden Teils (Fig. 2) zu erstrecken.

9. Absorbierender Artikel nach Anspruch 7, **dadurch gekennzeichnet, dass** das absorbierende Element (5) mit seinen Seitenkantteilen im Schrittbereich eine konkave Form bildet (Fig. 5).

10. Absorbierender Artikel nach irgendeinem der vorhergehenden Ansprüche, **dadurch gekennzeichnet, dass** die verbundenden Seitenkantteile (13, 15) und Endteile (14,16) des ersten und zweiten Stücks (1 bzw. 2) überlappend angeordnet sind, wobei die Innenseite eines überlappenden Teils gegen die Außenseite eines überlappten Teils angeordnet ist.

11. Absorbierender Artikel nach irgendeinem der vorhergehenden Ansprüche, **dadurch gekennzeichnet, dass** das zweite Stück (2) eine flüssigkeitsdurchlässige Innenschicht (4) und die flüssigkeitsundurchlässige Außenschicht (3) umfasst, und dass das absorbierende Element (5) zwischen der Innen- und der Außenschicht angeordnet ist, wobei sich die Innen- und die Außenschicht in der Seitenrichtung und Längsrichtung außerhalb des absorbierenden Elements (5) erstrecken und dort miteinander verbunden sind.

12. Absorbierender Artikel nach Anspruch 11, **dadurch gekennzeichnet, dass** die flüssigkeitsundurchlässige Außenschicht (3) nur das absorbierende Element deckt.

13. Absorbierender Artikel nach Anspruch 12, **dadurch gekennzeichnet, dass** der Rest des zweiten Stücks (2) atmungsaktiv und dampfdurchlässig ist.

14. Absorbierender Artikel nach Anspruch 11 oder 12, **dadurch gekennzeichnet, dass** die flüssigkeitsundurchlässige Außenschicht (3) atmungsaktiv und dampfdurchlässig ist, und dass der Rest des Artikels über seiner ganzen Fläche atmungsaktiv und dampfdurchlässig ist.

15. Absorbierender Artikel nach irgendeinem der vorhergehenden Ansprüche, **dadurch gekennzeichnet, dass** das erste Stück (1) rechteckig ist.

16. Absorbierender Artikel nach irgendeinem der vorhergehenden Ansprüche, **dadurch gekennzeichnet, dass** das zweite Stück (2) einen konkaven Teil (22) zwischen dem ersten Stück (1) und bis zu den Seitenkantteilen (14, 16) des zweiten Stücks (2) aufweist, wobei der konkave Teil Ausschnitte für die Beine des Benutzers bildet.

17. Absorbierender Artikel nach irgendeinem der vorhergehenden Ansprüche, **dadurch gekennzeichnet, dass** das zweite Stück (2) zumindest entlang einem Teil seiner Länge gegen das erste Stück (1) schmaler wird, wobei der eingeengte Teil Ausschnitte für die Beine des Benutzers bildet.

18. Absorbierender Artikel nach irgendeinem der vorhergehenden Ansprüche, **dadurch gekennzeichnet, dass** das zweite Stück (2) im Wesentlichen unelastisch ist.

19. Absorbierender Artikel nach irgendeinem der vorhergehenden Ansprüche, **dadurch gekennzeichnet, dass** elastische Elemente (23, 24) entlang den Kanten des zweiten Stücks (2), entlang mindestens dem Schrittteil für die Bildung von Beingummibänder angeordnet sind, die vorgesehen sind, um abdichtend um die Beine des Benutzers zu liegen.

20. Absorbierender Artikel nach Anspruch 19, **dadurch gekennzeichnet, dass** sich die elastischen Elemente (23, 24) in und von einem Bereich im Hinterteil, wo das erste (1) und zweite Stück (2) einander überlappen, und weiter entlang dem Schrittteil entlang den Kanten des zweiten Stücks in einer Richtung gegen den Vorderteil erstrecken.

21. Absorbierender Artikel nach irgendeinem der Ansprüche 4-20, **dadurch gekennzeichnet, dass** das absorbierende Element ein pH-regulierendes Material für die Regulierung des pH-Werts in der Umgebung um die Haut des Benutzers umfasst, wobei das pH-regulierende Material vorgesehen ist, um einen pH-Wert im Bereich 3,5-4,9 zu erreichen.

22. Verfahren zur Herstellung eines absorbierenden Artikel in Form einer nicht-wiederbefestigbaren absorbierenden Unterhose, wie z.B. einer Inkontinenzunterhose, einer Windelhose oder einer Bindeunterhose, wobei der absorbierende Artikel eine Längsrichtung und eine Querrichtung aufweist und einen elastischen Bauchteil, ein absorbierendes Element (5), das eine Länge und eine Breite aufweist und vorgesehen ist, um mindestens die Genitalien des Benutzers während der Anwendung des Artikels zu decken, und eine flüssigkeitsundurchlässige Außenschicht (3), der vorgesehen ist, um das absorbierende Element auf mindestens der Seite einzuschließen, die während der Anwendung des Artikels vom Benutzer abgewandt ist, umfasst
**dadurch gekennzeichnet,**
**dass** der elastische Bauchteil aus einem elastischen ersten Stück (1) hergestellt ist, das im Wesentlichen rechteckig ist und eine Länge und eine Breite aufweist und vorgesehen ist, um während der Anwendung teilweise den Torso des Benutzers zu umgeben und einen Hinterteil (8) und Seitenteile (9, 10) des unterhosenähnlichen Artikels zu bilden,
**dass** ein zweites Stück (2), das einen Teil des Artikels bildet, ausgeformt ist, um einen Vorderteil (11) und einen Schrittteil (12) des unterhosenähnlichen Artikels zu bilden, dass das zweite Stück (2) mit einer Länge und einer Breite hergestellt ist und mit seiner Längsrichtung entlang der Längsrichtung mit zwei gegenüberliegenden Endkanten und zwei gegenüberliegenden Längsseitenkanten angeordnet ist, dass die Breite des zweiten Stücks (2) zumindest im Schrittteil (12) kleiner als die Länge des ersten Stücks (1) ist, dass das zweite Stück mit seiner Längsrichtung in rechten Winkeln zur Längsrichtung des ersten Stücks angeordnet ist und an einem ersten Endteil (6) mit einem Kantteil (7) entlang der Länge des ersten Stücks, mittig darauf, verbunden ist,
**dass** ein Endteil (13) des ersten Stücks (1) mit einem ersten Seitenkantteil (14) von einer der Seitenkanten des zweiten Stücks (2) verbunden ist, und dass der andere Endteil (15) des ersten Stücks (1) auf entsprechender Art und Weise mit einem zweiten Seitenkantteil (16) von der anderen der Seitenkanten des zweiten Stücks verbunden ist, und wobei superabsorbierendes Material darüber hinaus im absorbierenden Element (5) angeordnet ist,
**dass** das absorbierende Element (5) mit einer Breite geformt ist, die in einem Bereich im Schritteil (12) am schmalsten ist, welcher Bereich während der Anwendung des Artikels eingerichtet ist, um im Wesentlichen vor den Genitalien des Benutzers angeordnet zu sein, dass das absorbierende Element mit einer Breite hergestellt ist, die in einer Richtung gegen diesen Bereich kontinuierlich schmaler wird, und dass superabsorbierendes Material im absorbierenden Element mit einer kontinuierlich zunehmenden Konzentration in der gleichen Richtung angeordnet ist, und dass das absorbierende Element (5) in seiner Gesamtheit auf dem zweiten Stück (2) angeordnet ist und Teil des Artikels bildet, bevor das Stück mit dem ersten Stück (1) verbunden wird.

## Revendications

1. Article absorbant dans la forme de culotte absorbante et non refixable, telle qu'une culotte d'incontinence, une culotte-couche ou une culotte serviette hygiénique, ledit article absorbant présentant une direction longitudinale et une direction transversale et comprenant une partie de taille élastique, un élément absorbant (5) qui présente une longueur et une largeur et est destiné à couvrir au moins les parties génitales de l'utilisateur pendant l'utilisation de l'article, et une couche extérieure imperméable aux liquides (3) qui est destinée à entourer l'élément absorbant sur au moins ce côté de celui-ci qui est détourné de l'utilisateur pendant l'utilisation de l'article,
**caractérisé en ce que**
ladite partie de taille élastique est faite à partir d'une première pièce élastique (1) qui présente une longueur et une largeur et qui est allongée dans une direction longitudinale et parallèle à ladite direction transversale et est destinée pendant l'utilisation à entourer partiellement le tronc de l'utilisateur et à former la partie arrière (8) et des parties latérales (9, 10) de l'article en forme de culotte,
**en ce qu'**une deuxième pièce (2) formant une partie de l'article est conçue pour former une partie avant (11) et une partie d'entrejambe (12) de l'article en forme de culotte, **en ce que** ladite deuxième pièce (2) présente une longueur et une largeur et est allongée le long de la direction longitudinale avec deux bords d'extrémité opposés et deux bords latéraux et longitudinaux opposés, **en ce que** la largeur de la deuxième pièce est, au moins dans la portion d'entrejambe (12), inférieure à la longueur de la première pièce (1),
**en ce que** la deuxième pièce (2) est agencée avec sa direction longitudinale à angles droits par rapport à la direction longitudinale de la première pièce et est reliée par une première partie d'extrémité (6) à une portion de bord (7) le long de la direction longitudinale de la première pièce, centralement sur celle-ci,
**en ce que** l'une partie d'extrémité (13) de la première pièce est reliée à une première portion de bord latérale (14) de l'un des bords latéraux de la deuxième pièce, et **en ce que** l'autre partie d'extrémité (15) de la première pièce est reliée d'une manière correspondante à une deuxième portion de bord latérale (16) de l'autre desdits bords latéraux de la deuxième pièce,
en outre, l'élément absorbant (5) dans sa totalité est disposé sur la deuxième pièce (2),
**en ce que** la largeur de l'élément absorbant (5) est la plus étroite dans une zone dans la partie d'entrejambe (12), zone qui, pendant l'utilisation de l'article, est conçue pour se trouver essentiellement en face des parties génitales de l'utilisateur, **en ce que** l'élément absorbant se rétrécit dans une direction vers ladite zone, et **en ce que** la capacité d'absorption de l'élément absorbant par unité de surface est la plus grande dans la zone la plus étroite.

2. Article absorbant selon la revendication 1, **caractérisé en ce que** ladite capacité d'absorption par unité de surface pour l'élément absorbant (5) augmente dans une direction vers l'espace le plus étroit.

3. Article absorbant selon la revendication 1 ou 2, **caractérisé en ce que** ladite capacité d'absorption par unité de surface pour l'élément absorbant (5) ne cesse de croître dans ladite direction.

4. Article absorbant selon l'une quelconque des revendications précédentes, **caractérisé en ce que** l'élément absorbant comprend un matériau superabsorbant, et **en ce que** la capacité d'absorption de l'élément absorbant (5) est commandée par le contenu du matériau superabsorbant.

5. Article absorbant selon l'une quelconque des revendications précédentes, **caractérisé en ce que** la longueur de la deuxième pièce (2) est supérieure à la largeur de la première pièce (1), et **en ce que** la partie en saillie de la deuxième pièce formée par la différence de longueur est dans sa totalité située au-dessous de ce bord latéral de la première pièce qui est la partie la plus basse pendant l'utilisation de l'article, et y forme la partie d'entrejambe (12).

6. Article absorbant selon la revendication 5, **caractérisé en ce que** ladite partie en saillie présente une largeur inférieure au reste de la deuxième pièce (2) (la figure 7).

7. Elément absorbant selon l'une quelconque des revendications précédentes, **caractérisé en ce que** l'élément absorbant (5) s'étend dans sa direction longitudinale sur toute la partie d'entrejambe (12) et un peu au-dessus de la partie avant (11) dans la direction dudit bord latéral de la deuxième pièce (2) qui est la partie la plus haute pendant l'utilisation de l'article.

8. Article absorbant selon l'une quelconque des revendications 1 à 6, **caractérisé en ce que** l'élément absorbant (5) est agencé de manière à s'étendre avec sa partie d'extrémité inférieure seulement un peu au-dessus de la partie d'entrejambe (12) et sur moins de la moitié de l'étendue de ladite partie en saillie (la figure 2).

9. Article absorbant selon la revendication 7, **caractérisé en ce que** l'élément absorbant (5) crée une forme concave avec ses portions de bord latérales dans la zone d'entrejambe (la figure 5).

10. Article absorbant selon l'une quelconque des revendications précédentes, **caractérisé en ce que** lesdites portions de bord latérales (13, 15) et parties d'extrémité (14, 16) reliées de ladite première et deuxième pièce (respectivement 1 et 2) sont disposées d'une manière chevauchante, avec l'intérieur d'une partie de chevauchement étant disposée contre l'extérieur d'une partie de chevauchement.

11. Article absorbant selon l'une quelconque des revendications précédentes, **caractérisé en ce que** la deuxième pièce (2) comprend une couche intérieure perméable aux liquides (4) et ladite couche extérieure imperméable aux liquides (3), et **en ce que** l'élément absorbant (5) est disposé entre lesdites couches intérieure et extérieure, les couches intérieure et extérieure s'étendant dans la direction latérale et la direction longitudinale à l'extérieur de l'élément absorbant (5) et étant y reliées l'une à l'autre.

12. Article absorbant selon la revendication 11, **caractérisé en ce que** la couche extérieure imperméable aux liquides (3) ne couvre que l'élément absorbant.

13. Article absorbant selon la revendication 12, **caractérisé en ce que** le reste de la deuxième pièce (2) est perméable à l'air et perméable à la vapeur.

14. Article absorbant selon la revendication 11 ou 12, **caractérisé en ce que** la couche extérieure imperméable aux liquides (3) est perméable à l'air et perméable à la vapeur et que le reste de l'article, sur toute sa surface, est perméable à l'air et perméable à la vapeur.

15. Article absorbant selon l'une quelconque des revendications précédentes, **caractérisé en ce que** la première pièce (1) est rectangulaire.

16. Article absorbant selon l'une quelconque des revendications précédentes, **caractérisé en ce que** la deuxième pièce (2) présente une partie concave (22) entre la première pièce (1) et jusqu'auxdites portions de bord latérales (14, 16) de la deuxième pièce (2), la partie concave formant des découpes pour les jambes de l'utilisateur.

17. Article absorbant selon l'une quelconque des revendications précédentes, **caractérisé en ce que** la deuxième pièce (2) est, au moins le long d'une partie de sa longueur, rétrécissant vers la première pièce (1), la partie rétrécissante formant des découpes pour les jambes de l'utilisateur.

18. Article absorbant selon l'une quelconque des revendications précédentes, **caractérisé en ce que** la deuxième pièce (2) est essentiellement inélastique.

19. Article absorbant selon l'une quelconque des revendications précédentes, **caractérisé en ce que** des éléments élastiques (23, 24) sont disposés le long des bords de la deuxième pièce (2), le long d'au moins la partie d'entrejambe pour la formation d'élastiques de jambe destinés à être en contact étanche avec les jambes de l'utilisateur.

20. Article absorbant selon la revendication 19, **caractérisé en ce que** les éléments élastiques (23, 24) s'étendent dans et depuis une zone dans la partie arrière dans laquelle la première (1) et la deuxième pièce (2) se chevauchent mutuellement et en outre le long de la partie d'entrejambe le long des bords de la deuxième pièce dans une direction vers la partie avant.

21. Article absorbant selon l'une quelconque des revendications 4 à 20, **caractérisé en ce que** ledit élément absorbant comprend un matériau de régulation de la valeur pH pour la régulation de la valeur pH dans l'environnement autour de la peau de l'utilisateur, le matériau de régulation de la valeur pH est agencé pour atteindre une valeur pH dans l'intervalle de 3,5 à 4,9.

22. Procédé de fabrication d'un article absorbant dans la forme de culotte absorbante et non refixable, telle qu'une culotte d'incontinence, une culotte-couche ou une couche serviette hygiénique, ledit article absorbant présentant une direction longitudinale et une direction transversale et comprenant une partie de taille élastique, un élément absorbant (5) qui présente une longueur et une largeur et est destiné à couvrir au moins les parties génitales de l'utilisateur pendant l'utilisation de l'article, et une couche extérieure imperméable aux liquides (3) qui est destinée à entourer l'élément absorbant sur au moins ce côté de celui-ci qui est détourné de l'utilisateur pendant l'utilisation de l'article,
**caractérisé en ce que**
ladite partie de taille élastique est faite à partir d'une première pièce élastique (1) qui est essentiellement rectangulaire avec une longueur et une largeur et est destinée pendant l'utilisation à entourer partiellement le tronc de l'utilisateur et à former une partie arrière (8) et des parties latérales (9, 10) de l'article en forme de culotte,
**en ce qu'**une deuxième pièce (2) formant une partie de l'article est conçue pour former une partie avant (11) et une partie d'entrejambe (12) de l'article en forme de culotte, **en ce que** ladite deuxième pièce (2) est pourvue d'une longueur et d'une largeur et est agencée avec sa direction longitudinale le long de la direction longitudinale avec deux bords d'extrémité opposés et deux bords latéraux et longitudinaux opposés, **en ce que** la largeur de la deuxième pièce (2) est conçue, au moins dans la partie d'entrejambe (12), inférieure à la longueur de la première pièce (1),
**en ce que** la deuxième pièce est agencée avec sa direction longitudinale à angles droits par rapport à la direction longitudinale de la première pièce et est reliée par une première partie d'extrémité (6) à une portion de bord (7) le long de la longueur de la première pièce, centralement sur celle-ci,
**en ce que** l'une partie d'extrémité (13) de la première pièce (1) est reliée à une première portion du bord latérale (14) de l'un des bords latéraux de la deuxième pièce (2), et **en ce que** l'autre partie d'extrémité (15) de la première pièce (1) est reliée d'une manière correspondante à une deuxième portion de bord latérale (16) de l'autre desdits bords latéraux de la deuxième pièce, et en outre, du matériau superabsorbant est disposé sur l'élément absorbant (5),
**en ce que** l'élément absorbant (5) est pourvu d'une largeur qui est la plus étroite dans une zone dans le partie d'entrejambe (12), zone qui, pendant l'utilisation de l'article, est conçue pour se trouver essentiellement en face des parties génitales de l'utilisateur,
**en ce que** l'élément absorbant est pourvu d'une largeur continuellement rétrécissante dans une direction vers ladite zone, **en ce que** du matériau superabsorbant est disposé dans l'élément absorbant avec une concentration ne cessant de croître dans la même direction, et **en ce que** l'élément absorbant (5) dans son intégralité est disposé sur la deuxième pièce (2) faisant partie de l'article avant que ladite pièce ne soit reliée à ladite première pièce (1).
